# EUROPEAN PATENT SPECIFICATION

(11) **EP 0 598 625 B1**
(45) Date of publication and mention of the grant of the patent: **13.01.1999**
(21) Application number: 93309238.9
(22) Date of filing: 19.11.1993
(51) Int. Cl.: A61F 5/448

(54) **Ostomy appliance**
Ostomievorrichtung
Dispositif d'ostomie

(30) Priority: 19.11.1992 GB 9224308
(43) Date of publication of application: 25.05.1994
(73) Proprietor: E.R. Squibb & Sons, Inc., Princeton, N.J. 08540-4000 (US)
(72) Inventor: Steer, Peter Leslie, East Grinstead, Sussex (GB)
(74) Representative: Holmes, Miles Keeton

(56) References cited:
- EP-A- 0 277 821
- US-A- 4 294 252
- US-A- 4 359 051
- US-A- 5 026 360

## Description

This invention relates to an ostomy appliance principally intended for use by obese persons who find it difficult or impossible to have a direct sight of the peristomal region to which an ostomy appliance is to be attached.

A proposal has been made, in U.S. Patent 4 359 051, inventor Oczkowski, that a hanger means be added to an ostomy appliance to facilitate proper alignment and mating of the coupling elements and for preventing accidental dropping of the collection bag. Moreover, the appliance is said to have means for maintaining positive seal while being worn, that is, mating cylindrical connectors on a base to be affixed to the ostomate and on the collection bag to be affixed to the base. These are said to be so proportioned that pressures created by body movements of the wearer tend to enhance rather than cause separation of the seal between the cylindrical connectors. The present invention provides a notable advance over Oczkowski's suggestion, and a simplification in design, in that the means of achieving proper alignment and coupling is an integral part of and is embodied in the structure of the body side coupling element. Moreover, Oczkowski's design is believed not to have been commercially successful.

An ostomy coupling as shown in EP-A-277821 of Craig Medical Products Limited has a pair of coupling rings. The body side ring has a flange attached to a medical grade adhesive pad and a chute wall surrounding a stomal aperture. The bag side ring has a rib member and a seal strip (20). To provide easy disengagement, the bag side ring has an integral flexible latching arm having a hook portion positioned to engage under a hook flange on the body side ring when the rings are coupled. The hook portion is disengagable from the hook flange by a lifting movement of the latching arm.

According to the present invention, there is provided an ostomy appliance having the features as defined in present claim 1.

The principal advantage of this arrangement is that once the adhesive pad is in place on the wearer, and the bag side coupling element is placed in the groove structure on the first leaf, the wearer can fold closed the second leaf onto the first leaf, and be assured that the first and second coupling elements are properly and sealingly mutually engaged. This closure, due to the hinging action of the plate-like folding member, can readily be done by the wearer without direct sight of the location of the appliance. To the best of the present inventors' knowledge and belief, no ostomy appliance has hitherto been available wherein joining of the bag coupling to the pad coupling can be readily and reliably achieved without sight of the peristomal area. To further assure the wearer that the appliance is properly attached and accurately closed, and that the bag will not become accidentally detached, a latching arrangement is provided. In a preferred embodiment of the present invention, the latching arrangement comprises a detent tab on the second coupling element which co-operates with a portion of the distal end of the first leaf, this portion acting as counterpart detent tab.

In a preferred embodiment of the invention, both the plate-like folding member and the first coupling element on the bag may be made of a synthetic plastics material.

The invention will be better understood from the following non-limiting description of a example thereof given with reference to the accompanying drawings, in which:-
Figure 1 is a front view of the plate-like folding member shown in its closed condition;
Figure 2 is a front view similar to Figure 1 but showing the member in its open or unfolded position;
Figure 3 is a vertical cross-section, taken on the centre line of the plate-like member, showing it part-open; this Figure also shows a bag with a first coupling element thereon about to be lodged in the slot structure provided on the first leaf of the plate-like member;
Figure 4 is a cross section taken in a vertical plane showing the parts in their closed condition, corresponding to Figure 1; and
Figure 5 is a view half in cross-section taken on the line A-A of Figure 4 and half in side elevation and also shows the parts in the closed condition.

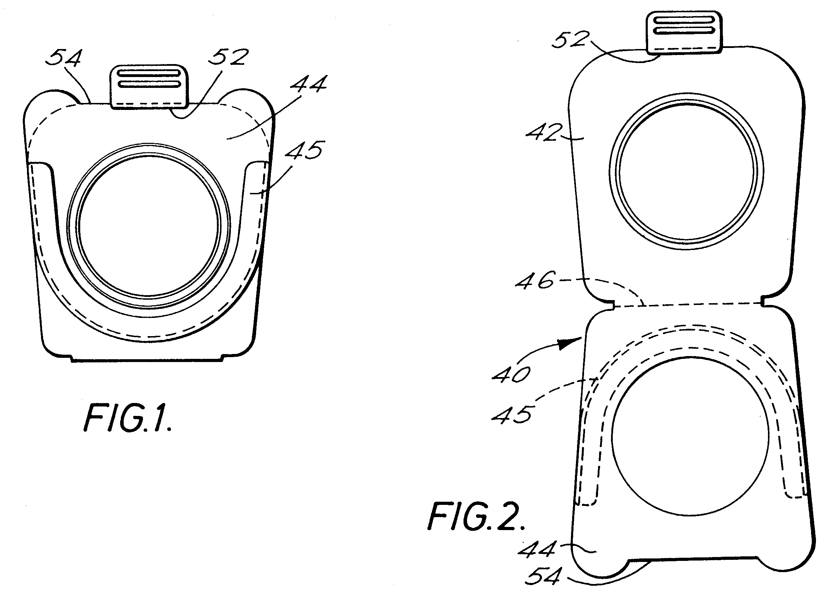

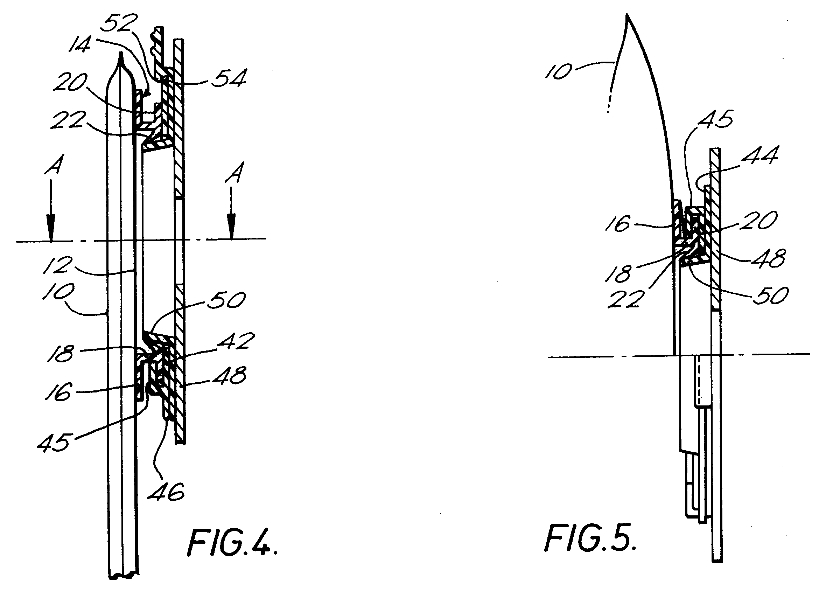

Referring firstly to Figures 1-5, the illustrated appliance includes a bag 10 (Figs. 4 and 5) having a stomal orifice 12, to which is attached a first coupling element 14. The element 14 is made of a synthetic plastics material and comprises a substantially flat flange 16, a ring-like wall 18 and a generally annular flange 20. Extending radially inwardly, from the inner edge of flange 20, is a flexible deflectible sealing strip 22 which extends completely around the stomal orifice defined by the encircling wall 18. The bag 10, which is usually made of a pair of sheets of plastics film suitably sealed around their periphery, is fixed to the left hand side surface (as seen in Figs. 4 and 5) of the flange 16 in any suitable way, for example by heat welding, RF welding or adhesive.

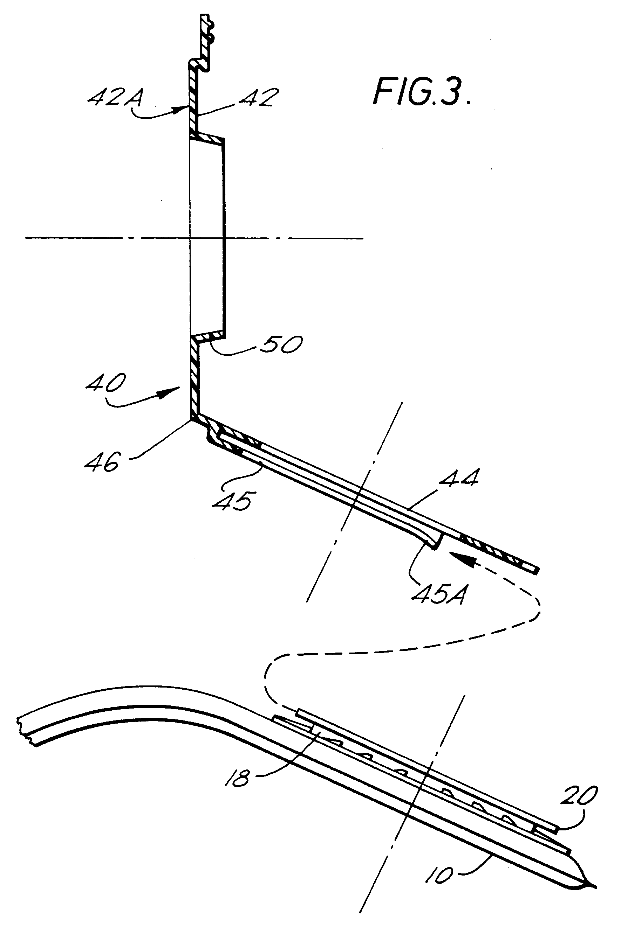

The second (also called body side) coupling element is seen best from Figures 1-3 and takes the form of a plate-like folding member having two leaves, of which the first leaf is provided with a groove structure for receiving the first (bag side) coupling element. The plate-like folding member 40 has a first leaf 42 and a second leaf 44. The leaf 44 has projecting therefrom a structure 45 which is L-shaped as seen in section and which curves around in an approximately U-shape as seen in elevation (Fig. 2). The purpose of this L-shaped wall, which defines a groove structure, is to receive the flange 20 of the bag side coupling element. The two leaves 42 and 44 are hinged together by a plastics hinge 46, as seen in Figures 2, 3 and 4. As seen in Figure 3, the wall 45, which is L-shaped as seen in the cross sectional view of Figure 4, has the two ends of the "U" formation angled slightly outwardly (as seen at 45A in Fig. 3) in order to provide an entry lead-in, facilitating insertion of the bag side coupling element into the groove defined by the structure 45. The leaf 42 includes a flat annular portion having a surface 42A to which is fixed, in any suitable manner, a pad 48, seen in Figures 4 and 5 only, of medical grade adhesive material. Suitable adhesive materials for this purpose include, for example, those described by Chen in U.S. Patent 3,339,546; by Chen et al. in U.S. Patent No. 4,192,785; by Pawelchak et al. in U.S. Patent No. 4,393080; and by Doyle et al. in U.S. Patent No. 4,551,490. A particularly suitable material is that sold under the Registered Trade Mark STOMAHESIVE by Bristol-Myers Squibb of Ickenham, Middlesex. As is conventional in ostomy couplings, provision may be made on the free surface of the medical grade adhesive pad for markings directed to assisting the user to cut out a central portion of the pad, in registry with the stomal orifice.

The pad 48 has a stomal orifice. The leaf 42 also has a shallow tubular protrusion 50 therefrom, having a wall tapering inwardly at a small angle, for example 10°. The wall is also shown at 50 in Figures 4 and 5. The outside diameter of the wall 50 at its inner or minimum diameter portion and its taper are chosen so that the wall 50 makes a good sealing fit with the sealing strip 22 of the bag side coupling element. In this way, the mouth of the space defined by the annular wall 50 is brought closely adjacent to and in registry with the stomal orifice 12 in the bag 10. As will be understood, this is achieved by folding over the leaf 44 by a raising motion, this being done after the bag side coupling element 20 has been snugly inserted within the groove structure 45.

In order to latch the leaf member 44 closed, a latching detent 52 is provided on the leaf 42 of the body side coupling element. This detent 52 engages with a portion 54 (Fig. 2) of the edge of the leaf 44. This detent and surface are seen in the engaged position at the top of Figure 4.

It will be seen that because of the provision of a body side coupling element in the form of a hinged foldable member, the path of movement of the bag side coupling element into engagement with the body side coupling element is an arcuate movement predetermined by the folding action, and the wearer of the appliance can therefore be secure in the knowledge that the bag side coupling element has been brought into proper aligned engagement with the body side coupling element and that the two parts are held together reliably by the detent arrangement 52, 54. Consequently, it is no disadvantage for an obese wearer of this appliance to be unable to see the peristomal area where it is applied, since by manipulation of the folding action he or she can be assured of a proper joining of the bag and body side coupling elements.

## Claims

1. An ostomy appliance having a first coupling element (14) attached to an ostomy bag (10) and a second coupling element (40) attached to a medical grade adhesive pad (48), the second coupling element 40 includes a plate-like folding member having two leaves (42, 44) hinged together of which the second leaf (44) comprises a tubular conduit member (50) on one side thereof, the other side of the leaf being provided with the said pad of medical grade adhesive material, a flange (20) on the first coupling element (14) carrying a flexible deflectable sealing strip (22) and which is engagable with the conduit member (50) in a sealing manner in the folded closed position of the leaves characterised in that, the first leaf of the second coupling element has a groove structure (45) for receiving an edge portion of the flange (20) of the first coupling element (14); the tubular conduit member being tapered, there being a latching arrangement (52, 54) on the leaves for holding the leaves closed.

2. An appliance according to claim 1 in which the latching arrangement comprises a detent tab (52) on the second coupling element (40) which co-operates with a portion of the distal end of the first leaf (44), this portion acting as counterpart detent tab (54).

3. An appliance according to claim 1 or 2 in which both the plate-like folding member (42, 44) and the first coupling element (14) are made of a synthetic plastics material.

4. An appliance according to claim 1, 2 or 3, in which the groove structure (45) takes the form of an approximately U-shaped wall which is L-shaped as seen in cross-section.

## Patentansprüche

1. Stomavorrichtung mit einem an einem Stomabeutel (10) angebrachten ersten Kupplungselement (14) und einem an einer medizinischen Klebekompresse (48) angebrachten zweiten Kupplungselement (40), wobei das Zweite Kupplungselement 40 ein plattenartiges Faltglied einschließt, das zwei Blätter (42, 44) aufweist, die gelenkig verbunden sind, von denen das zweite Blatt (44) ein rohrförmiges Leitungsglied (50) an einer Seite davon aufweist, wobei die andere Seite des Blattes mit der Kompresse aus medizinischem Klebematerial versehen ist, ein Flansch (20) am ersten Kupplungselement (14) einen flexiblen ablenkbaren Dichtungsstreifen (22) trägt, der mit dem Leitungsglied (50) auf eine dichtende Weise in der gefalteten geschlossenen Position der Blätter eingreiffähig ist, dadurch gekennzeichnet, daß das erste Blatt des zweiten Kupplungselementes eine Nutstruktur (45) zum Aufnehmen eines Kantenabschnittes des Flansches (20) des ersten Kupplungselementes (14) aufweist; wobei das rohrförmige Leitungsglied verjüngt ist, wobei es einen Klinkenmechanismus (52, 54) an den Blättern gibt, um die Blätter geschlossen zu halten.

2. Vorrichtung nach Anspruch 1, in welcher der Einrastmechanismus eine Arretierungslasche (52) am zweiten Kupplungselement (40) aufweist, die mit einem Abschnitt des distalen Endes des ersten Blattes (44) zusammenwirkt, wobei dieser Abschnitt als Gegenstück-Arretierungslasche (54) dient.

3. Vorrichtung nach Anspruch 1 oder 2, in der sowohl das plattenartige Faltglied (42, 44) als auch das erste Kupplungselement (14) aus einem Kunststoffmaterial bestehen.

4. Vorrichtung nach Anspruch 1, 2 oder 3, in der die Nutstruktur (45) die Form einer näherungsweise U-förmigen Wand annimmt, die im Querschnitt betrachtet L-förmig ist.

## Revendications

1. Dispositif de stomie comportant un premier élément de raccord (14) fixé à une poche de stomie (10) et un second élément de raccord (40) fixé à un tampon adhésif (48) de qualité médicale, le second élément de raccord (40) comportant un élément pliant en forme de plaque avec deux rabats (42, 44) articulés l'un sur l'autre, le second rabat (44) comprenant un élément de conduite tubulaire (50) sur l'un de ses côtés, l'autre côté de ce rabat étant muni dudit tampon de matière adhésive de qualité médicale, une collerette (20), sur le premier élément de raccord (14), portant une bande d'étanchéité (22) souple et flexible et qui peut s'engager sur l'élément de conduite (50) de manière étanche, dans la position repliée et fermée des rabats, caractérisé en ce que le premier rabat du second élément de raccord comporte une structure de gorge (45) destinée à recevoir un bord de la collerette (20) du premier élément de raccord (14); l'élément de conduite tubulaire étant conique, un dispositif de blocage (52, 54) étant prévu sur les rabats pour les maintenir fermés.

2. Dispositif selon la revendication 1, dans lequel le dispositif de blocage comprend une patte de détente (52), sur le second élément de raccord (40), qui coopère avec une partie de l'extrémité distale du premier rabat (44), cette partie jouant le rôle d'une patte de détente complémentaire (54).

3. Dispositif selon la revendication 1 ou 2, dans lequel aussi bien l'élément pliant en forme de plaque (42, 44) que le premier élément de raccord (14) sont faits d'une matière plastique synthétique.

4. Dispositif selon la revendication 1, 2 ou 3, dans lequel la structure de gorge (45) prend la forme d'une paroi en forme approximative de U qui, vue en coupe, est en forme de L.
